# EUROPEAN PATENT APPLICATION

(11) **EP 3 287 103 A1**
(43) Date of publication of application: **28.02.2018**
(21) Application number: 16185110.0
(22) Date of filing: 22.08.2016
(51) Int. Cl.: A61F 5/01, A61F 13/10

(54) **WRIST BRACE**

(71) Applicant: Njoy Braces B.V., 3235 CD Rockanje (NL)
(72) Inventor: Brand, Johanna Louise, 3235 CD Rockanje (NL); Vermeulen, Jacobus Johannes Waltherus, 3235 CD Rockanje (NL)
(74) Representative: van der Krans, Arie

(57) **Abstract**

A wrist brace for supporting a subject's wrist, comprising a lower arm support section for circumferentially enclosing a subject's lower arm section, a hand support section for circumferentially enclosing a metacarpal section of the subject's hand, a connecting member for connecting the lower arm support section and the hand support section of the brace. The lower arm support section, the hand support section and the connecting member are formed as interconnected dimensionally stable parts having an inner surface and an outer surface, based on a model of the lower arm wrist and hand and wherein the dimensions are chosen to provide a margin between an outline of the lower arm wrist and hand of the model and an inner surface of the wrist brace.

## Description

### FIELD OF THE INVENTION

The invention relates to a wrist brace for providing support for a subject 's wrist and a method of manufacturing the wrist brace.

### BACKGROUND

A wrist brace limits the mobility of segments of the musculoskeletal system in order to protect anatomical structures and to combat or prevent pain or to provide or maintain a better functional starting point than the musculoskeletal system itself can generate. The pain or functional restriction may find it's origin in local diseases of the joints, muscles, tendons, nerves, skin or the subcutaneous tissue. The tissues involved can be affected by illness, wear, trauma, overload, crushing, pinching, hypermobility or other causes.

By limiting the movement locally, all of said structures may be protected or relieved and be brought in a functionally good starting position.

Wrist braces or wrist-thumb braces are manufactured as individual facility custom-made device or may be available as a ready-made device. Features of the brace may be customized. Such features are largely manufactured manually.

The wrist brace rests at one end on the subject's lower arm. From the lower arm, the wrist brace extends towards the subject's hand, where support elements provide support for the wrist, hand and one or more fingers, such as the thumb in a wrist-thumb support.

Wrist braces in the art are usually manufactured by means of positive and negative models, for example gypsum models of the arm and wrist, which are manually corrected. The materials which may be used in wrist braces include leather, plastic, fabric or elastics and usually with local stiffeners. To date, an enclosure of the anatomical segment is made. This enclosure is open in a longitudinal direction and can be opened in order to lay the arm / hand in place and is thereafter closed by a fastener. The fastener may take any form, Velcro is typically used. The enclosure may be perforated to prevent perspiration.

Braces in the art have disadvantages. The shape of the wrist braces as described is not stable; this is dependent on the extent to which the fasteners are tightened. Thus the amount of support of the brace is not stable. Putting on or taking off may be difficult for one who has to wear the brace; this person has only one hand, the one which is not supported, available to open the brace, to get the affected arm / hand in place and handle several Velcro straps which often adhere to themselves and to objects in the environment.

The Velcro eventually gets contaminated and weak. The Velcro straps thicken the brace and are hard to combine with putting on and taking off of regular clothing.

With wrist braces in the art, users often complain about perspiration, which may lead to softening of the skin which may cause medical problems such as wounds, infections and eczema.

Cleaning of the conventional brace is often difficult due to the choice of materials, stitching and difficult to clean Velcro. When the braces having textile parts are wet, drying may require a prolonged period. Subsequently the braces are often taken off when visiting toilets and other wet activities such as showering, washing or window cleaning. As a result, the wearer lacks the protection of the brace in activities which are burdensome, just when protection is required.

Moreover, often the conventional brace should in the course of the day be adjusted several times in response to swelling of the wrist-hand-arm, depending on the wrist condition, the environmental temperature and biorhythm or other circumstances.

### SUMMARY

It is an object of the invention to provide a wrist brace which overcomes the above mentioned problems and/or disadvantages.

The object is achieved in a wrist brace for supporting a subject's wrist, comprising an lower arm support section for circumferentially enclosing a subject's lower arm section, a hand support section for circumferentially enclosing a metacarpal section of the subject's hand, a connecting member for connecting the lower arm support section and the hand support section of the brace. The lower arm support section, the hand support section and the connecting member are formed as interconnected dimensionally stable parts having an inner surface and an outer surface, based on a model of the lower arm, wrist and hand and wherein the dimensions are chosen to provide a margin between an outline of the lower arm, wrist and hand of the model and an inner surface of the wrist brace.

By forming the wrist brace as dimensionally stable interconnected parts, a dimensionally stable wrist brace is formed. Movement of the wrist is counteracted, thus providing the required support for the wrist. The space between the outline of the lower arm, wrist and hand and inner surface of the brace allows the brace to be put on and taken off easily. Moreover, any swelling of the lower arm, wrist or hand can be sustained by the space between brace and arm.

The construction requires no fasteners, thereby allowing the brace to be put on and taken off with the free hand only. The integrated construction allows a smooth surface and requires no seams, stitches or material transitions, which allows hygienic use of the brace. The brace can be kept on while engaging in wet activities such as showering or toilet use while maintaining the protective function, as the brace is insensitive to water.

In an embodiment, the wrist brace is formed as an integrally connected solid part in one piece. This allows the wrist brace to be manufactured using additive manufacturing technologies such as for example fused deposition modelling or selective laser sintering. An advantage of this is that no further assembly of the wrist brace is required. Once produced, apart from some deburring and the like, it is ready for use without further redo.

In an embodiment, the hand support section and the lower arm support section are formed as circumferentially enclosing parts, enclosing corresponding metacarpal part of the hand and lower arm respectively.

The hand support section and lower arm support section are arranged parallel to the subject's skin.

In an embodiment, the connecting member has at least two longitudinal connecting members extending from the lower arm support section to the hand support section and at least one cross member connecting the at least two longitudinal connecting members.

In an embodiment, the wrist brace further comprises at least one elongated slot adjacent to one of the at least one connecting members. The space between the longitudinal members provides additional space for the hand when putting on or taking off of the brace. Especially the thumb portion of the hand is accommodated. Moreover the space between the longitudinal members provide further improved ventilation.

In an embodiment, the at least one cross member connecting the at least two longitudinal connecting members.

The cross member provides enhanced structural strength of the brace.

The at least one cross member defines openings between the at least two longitudinal connecting members and lower arm support section and hand support section.

In an embodiment, the wrist brace further comprises a thumb support connected to the connecting member, the thumb support comprising a semi-circular or cuff-shaped or annular element. The thumb support can be made as an extension of the hand support section.

In an embodiment, the wrist brace further comprises a finger support extending from the hand support section, the finger support comprises a curved surface extending from the hand support section at a hand palm section of the hand support section. Like the thumb support, the finger support can be made as an extension of the hand support section.

In an embodiment, the hand support section has an extended section.

The extended hand support section can be used for supporting one or more fingers. The width and location of the extended section can be adapted in accordance with the location and number of fingers to be supported.

In an embodiment, the at least one longitudinal connecting member is provided with openings. This allows better ventilation and enhances the hygienic use of the brace. Perspiration fluid is easily ventilated from the brace.

The object is further achieved in a method of manufacturing a wrist brace as described above. The method comprises capturing a three dimensional image of the subject's lower arm and hand, defining an arm support area and a hand support area from the three dimensional image of the subject's lower arm, calculating a three dimensional model of the wrist brace from the three dimensional image of the subject's lower arm, the three dimensional model defining the lower arm support section and the hand support section at the defined arm support area and the hand support area, and defining a connecting member in the three dimensional model of the wrist brace. This way the brace can be manufactured without a need for making a mould or physical model, thus significantly reducing manufacturing time and labour.

The method further comprises determining a cross section shape of the lower arm support section and a cross-sectional shape of the hand support section from the three dimensional model, determining dimensions of the cross-sectional of the lower arm support section and dimensions of the cross section of the hand support section in accordance with the cross sections of corresponding zones of the three dimensional model increased with a predetermined margin.

This allows a tolerance margin between the subject's lower arm skin and the inner side of the brace.

The method further comprises manufacturing the wrist brace from the three dimensional model.

In an embodiment, the method of manufacturing the wrist brace from the three dimensional model comprises additive manufacturing the wrist brace.

### BRIEF DESCRIPTION OF THE DRAWINGS

Fig. 1 a shows a schematic overview of a wrist brace according to an embodiment of the invention.
Fig. 1b shows a schematic overview of a connecting area for the wrist brace of fig. 1 a according to an embodiment of the invention.
Fig. 2a - 2c show an isometric view from various viewing angles of the wrist brace according to an embodiment of the invention.
Fig. 3 shows an isometric view of the wrist brace having a finger support section according to an embodiment of the invention.
Fig. 4a shows an isometric view of the wrist brace having a thumb support section according to an embodiment of the invention.
Fig. 4b shows another isometric view of the wrist brace having the thumb support section according to an embodiment of the invention.

### DETAILED DESCRIPTION OF EMBODIMENTS

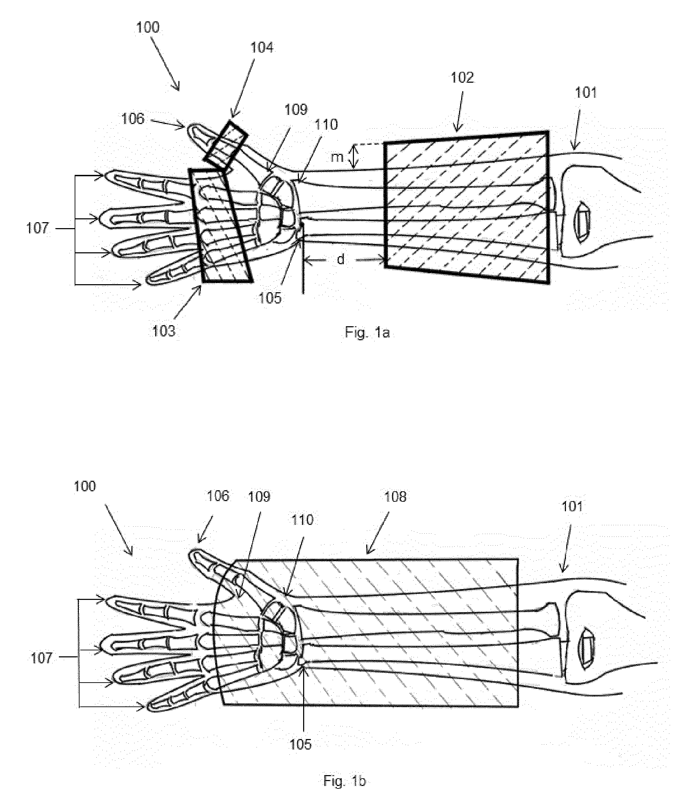

In fig. 1 a a wrist brace 100 is shown schematically having support areas 102-104 for supporting a subject's wrist 110 located between the subject lower arm 101 and hand 109. The wrist 110 can be supported by a lower arm support 102 and a hand support section 103, and optionally a thumb support 104 which can be interconnected connected by a connecting member 108 as schematically shown in fig. 1 b.

When the lower arm support section 102, and hand support section 103 are interconnected by the connecting member 108, the wrist 110 is put into a fixed resting position.

In fig. 1a, a minimal distance 'd' is shown between the lower arm support 102 and the caput ulnae 105. This can normally be a distance in a range of 5-20 centimeters.

Furthermore a margin 'm' is shown between the inner diameter of the arm support 102 and the outer circumference of the arm 101. The margin provides ventilation between the arm support and lower arm 101, while maintaining the support and fixative function for the wrist 110. Similar margins apply to the hand support section 103 and thumb support shown in fig. 1 a. The connecting member 108 may have other margins than the margins of the arm support 102, hand support section 103 and thumb support 104. The margin 'm' can normally be chosen in a range of 0-1.5 cm.

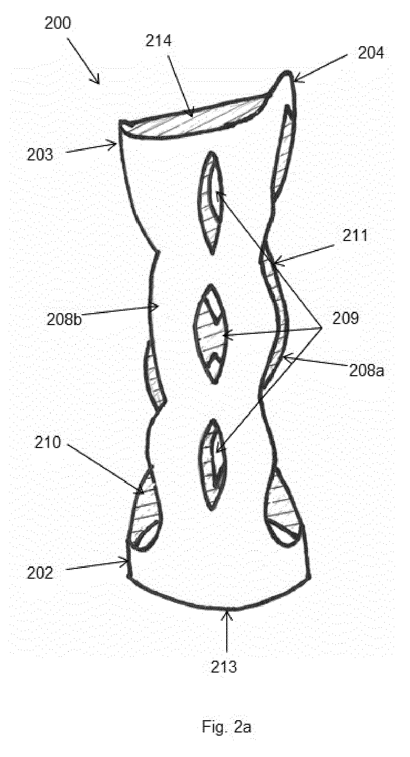

In fig. 2a a right hand wrist brace 200 is shown having a lower arm support section 202, corresponding to the lower arm support section 102 of fig. 1a, a hand support section 203 corresponding to the hand support section 103 of fig. 1 a. The wrist brace 200 has in this example two interconnecting members 208a, 208b for interconnecting the lower arm support section 202 and the hand support section 203. A single interconnecting member 208 may apply, as well as more than two interconnecting members. The interconnecting members 208a, 208b can comprise a dorsal interconnecting member 208a and a palmar interconnecting member 208a, referring to the respective back and palm of a hand introduced into the wrist brace 200 as shown in figures 2a - 2c.

Openings 209 in the interconnecting members 208a, 208b provide ventilation for the enclosed lower arm 101 and wrist 110, while at the same time making the construction of the wrist brace 200 lighter without loss of structural strength. Moreover, the openings 209 may be patterned as to create an esthetic effect.

An apex 204 of the hand support section 203 indicates an extension of the hand support section 203 creating a resting position for a subject's forefinger.

In fig. 2a two elongated slots 210, 211 are shown between the connection members 208a, 208b, the lower arm support section 202 and the hand support section 203. The lower arm support section 202 has an opening 213 for inserting subject's hand with the back of the hand facing the inside of the dorsal interconnection member 208a. The elongated slot 211 operates as a thumb guide, allowing the thumb to pass from the lower arm support section 202 to the hand support section 203 when the user's hand is inserted in the wrist brace 200 through opening 213.

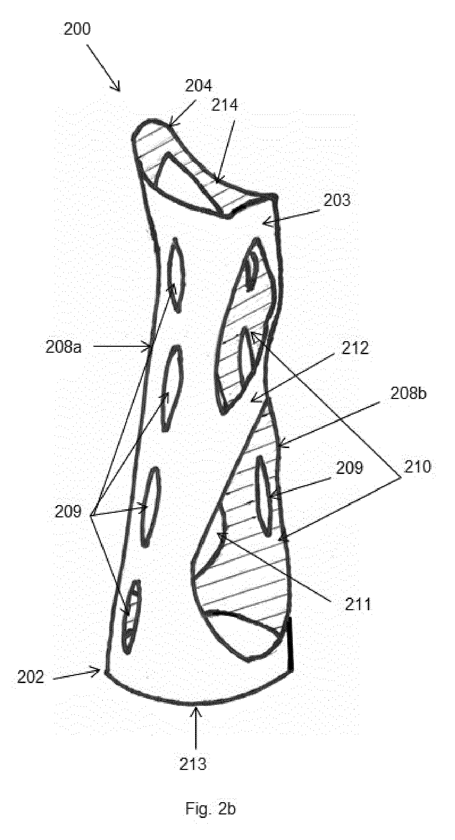

In fig. 2b the wrist brace 200 is shown from another viewing angle. A cross member 212 can be arranged across opening 210 between the connecting members 208a and 208b. This can reinforce the structural stability of the wrist brace 200. By arranging the cross member 212 in for example an oblique manner, also an esthetic effect may be achieved.

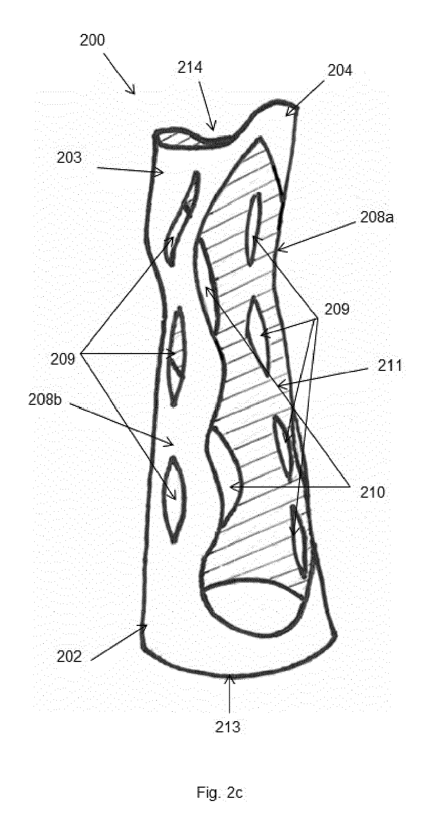

In fig. 2c, the wrist brace 200 is shown from a opposite viewing angle as in fig. 2b. The elongated slot 211 is shown facing the viewer. After inserting the subject's hand through opening 214 of the hand support, the thumb 306 can rest against the outside of apex 204 of the hand support section 203, while the fore finger may rest against the inside of the apex 204.

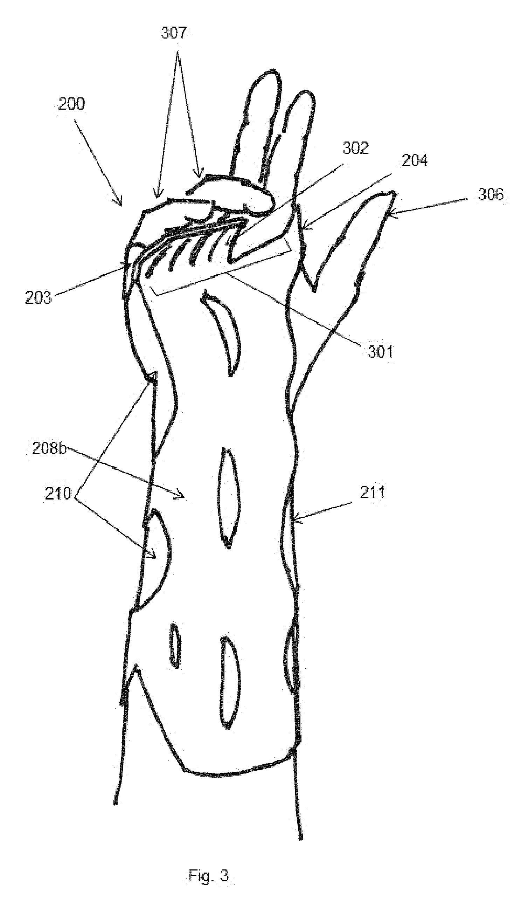

Fig. 3 shows the wrist brace 200 of figures 2a - 2c, wherein a hand palm section 301 of the hand support section 203, a finger support 302 is arranged for providing a support for the two fingers 307 shown in the example of fig. 3. The skilled person will understand that by adapting the width and location within the hand palm section 301 of the hand support section 203 of the finger support 302 more or less fingers 307 may be supported.

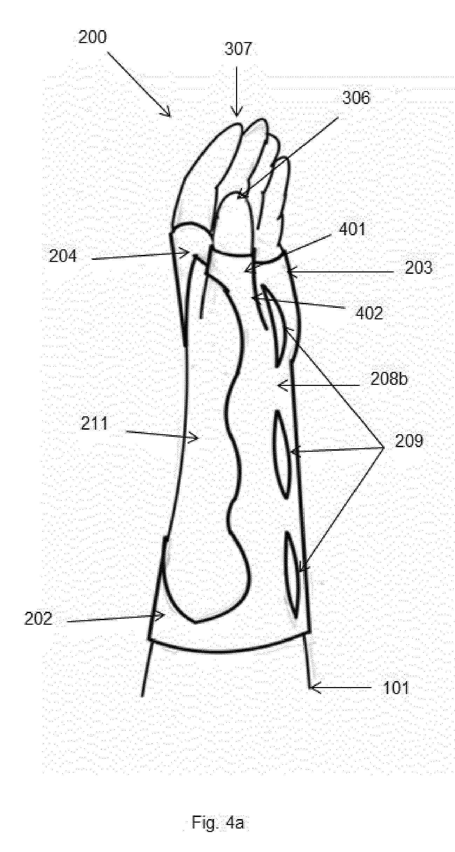

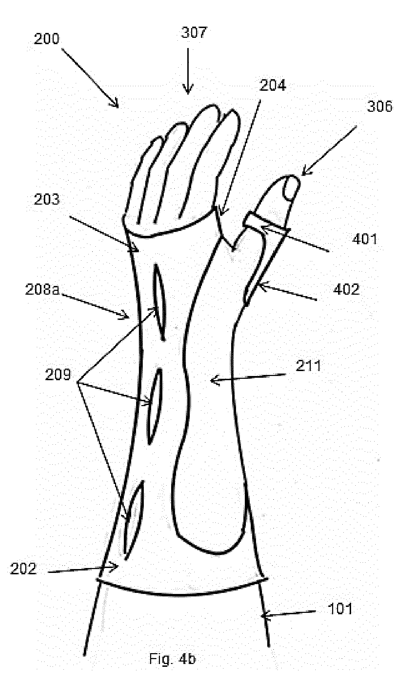

In fig. 4a, a left hand wrist brace 200 is shown having a thumb support 401. The thumb support 401 is connected to the palmar connecting member 208b and/or the hand support section 203 via an extension of the palmar interconnecting member 208b as interconnecting part 402. Fig. 4b shows the wrist brace from another viewing angle.

The thumb support 401 can comprise an annular or cuff-shaped element for encircling and supporting the thumb 306, having an inner diameter adjusted to the thumb diameter increased with a margin m as described. When introducing the subject's hand in the wrist brace at the lower arm support section side in opening 213, the thumb 306 extends through the elongated opening 211, acting as a thumb guide. The thumb 306 now can be introduced into the annular or cuff-shaped thumb support 401, while the fingers 307 and hand extend through the hand support opening 214.

The wrist brace 200 has a rigidity depending on the truss structure of the connecting members and optional cross members. Also a choice of materials will determine the strength and rigidity of the wrist brace 200. When putting on the wrist brace, or taking it off, a certain resiliency of the brace can be desired. Preferably any deformation of the brace is elastic, causing any compressed parts to return to their original position and shape, when a force to such part is applied during putting on or taking off, and which force is subsequently removed.

The wrist brace 200 can be manufactured on the basis of the 3D module of the lower arm, wrist and hand. From the 3D module, inner dimensions of the brace are determined with a margin for allowing the lower arm, wrist and hand to have a certain freedom of movement within the brace. The margin however is chosen such that the wrist is sufficiently supported and fixed within the wrist brace 200.

The wrist brace 200 as described above can be manufactured as an integral object using additive production techniques, such as fused deposition modeling (FDM) or 3D printing technologies, and also using selecting laser sintering (SLS) or stereo lithography (SLA). These technologies are also known as rapid prototyping technologies, which are ultimately suitable for the production of one-off objects, such as the wrist brace.

Materials suitable for additive manufacturing the wrist brace 200 include thermoplastic materials including PLA and Nylon, but also a metal may apply (using SLS techniques for example).

The method of manufacturing can be performed using camera's for capturing the three dimensional image of the subject's lower arm and computer such as a personal computer, of a control system having a processor such as a microprocessor and a memory to perform the necessary calculations and for determining the three dimensional model of the arm and for determining the three dimensional model of the wrist brace having the necessary dimensions and margins as described. The skilled person is aware that a wrist brace can be manufactured in additive manufacturing or rapid prototyping technologies using the three dimensional model of the wrist brace.

The embodiments above are given by a way of example only. The features of the described embodiments of the wrist brace apply interchangeably to both left and right arm wrist braces. Changes and deviations can be made from these examples, without departing from the scope of the claims set out below.

### REFERENCE NUMERALS

- 100: schematic overview of a wrist brace
- 101: lower arm
- 102: lower arm support
- 103: hand support
- 104: thumb support area
- 105: caput ulnae
- 106: thumb
- 107: fingers
- 108: connection area
- 109: hand
- 110: wrist
- 200: wrist brace
- 202: lower arm support section
- 203: hand support section
- 204: apex
- 208a: dorsal connecting member
- 208b: palmar connecting member
- 209: ventilation opening
- 210: elongated slot
- 211: elongated slot
- 212: cross member
- 213: arm support opening
- 214: hand support opening
- 301: hand palm section
- 302: finger support section
- 306: thumb
- 307: fingers
- 401: thumb support
- 402: connecting member for thumb support

## Claims

1. Wrist brace (100, 200) for supporting a subject wrist, comprising
• an lower arm support section (102, 202) for circumferentially enclosing a subject's lower arm section;
• an hand support section (103, 203) for circumferentially enclosing a metacarpal section of the subject's hand;
• at least one connecting member (108, 208a, 208b) for connecting the lower arm support section (102, 202) and the hand support section (103, 203) of the wrist brace (100, 200);
**characterized by**
• the lower arm support section (102, 202), the hand support section (103, 203) and the at least one connecting member 208 and/or thumb support (401) and/or finger support (302 are interconnected dimensionally stable parts having inner dimensions based on dimensions of the lower arm, wrist and hand increased with a margin to provide a space between an outline and an inner surface of the wrist brace.

2. Wrist brace (100, 200) according to claim 1, wherein the wrist brace is formed as integrally connected solid parts in one piece.

3. Wrist brace (100, 200) according to any of the preceding claims, wherein the hand support section (103, 203) and the lower arm support section (102, 202) are formed as circumferentially enclosing parts, enclosing corresponding parts of the hand (109) and lower arm (101).

4. Wrist brace (100, 200) according to any of the preceding claims, wherein the at least one connecting member (108, 208a, 208b) comprises at least two longitudinal connecting members (208a, 208b) extending from the lower arm support section (102, 202) to the hand support section (103, 203).

5. Wrist brace according to claim 5, further comprising at least one cross member (212) connecting the at least two connecting members (208a, 208b).

6. Wrist brace according to any of the preceding claims, further comprising at least one elongated slot (210, 211) adjacent to one of the at least one connecting members (208a, 208b).

7. Wrist brace (100, 200) according to any of the preceding claims, further comprising a thumb support (401) connected to the connecting member (108, 208a, 208b) via thumb support connecting member (402), the thumb support (401) comprising a semi-circular or cuff -shaped element for enclosing the thumb (306).

8. Wrist brace (100, 200) according to any of the preceding claims, further comprising a finger support section (302) extending from the hand support section (103, 203), the finger support section (302) comprising a curved surface extending from the hand support section (103, 203) at a hand palm section of the hand support section (103, 203).

9. Wrist brace (100, 200) according to any of the preceding claims, wherein at least one of the connecting members (208a, 208b), the lower arm support section (102, 202) and the hand support section (103, 203) is provided with openings (209).

10. Method of manufacturing a wrist brace (100, 200) according to any of the preceding claims 1 - 9 , the method comprising
• capturing a three dimensional image of the subject's lower arm (101), wrist (110) and hand (109);
• calculating a three dimensional model of the wrist brace (100, 200) from the three dimensional image of the subject's lower arm, wrist and hand, the three dimensional model defining an lower arm support section (102, 202), a hand support section (103, 203) and at least one connecting member (108, 208a, 208b) in the three dimensional model of the wrist brace (100, 200);
• determining a shape of a cross section of the lower arm support section (102, 202) and a shape of a cross section of the hand support section (103, 203) from the three dimensional model;
• determining dimensions of the cross section of the lower arm support section (102, 202) and dimensions of the cross section of the hand support section (103, 203) in accordance with the cross sections of corresponding zones of the three dimensional model increased with a predetermined margin, to allow a tolerance between the subject's lower arm skin and the inner side of the wrist brace (100, 200);
• manufacturing the wrist brace (100, 200) from the three dimensional model.

11. Method of manufacturing a wrist brace (100, 200) according to claim 10, wherein the manufacturing of the wrist brace (100, 200) from the three dimensional model comprises additive manufacturing the wrist brace (100, 200).
